(19) Europäisches Patentamt / European Patent Office / Office européen des brevets

(11) **EP 0 877 033 B1**

(12) **EUROPEAN PATENT SPECIFICATION**

(45) Date of publication and mention
of the grant of the patent:
**12.09.2007   Bulletin 2007/37**

(51) Int Cl.:
*C08F 2/32* [(2006.01)]     *A61K 9/51* [(2006.01)]

(21) Application number: **97303127.1**

(22) Date of filing: **08.05.1997**

(54) **A process for the preparation of highly monodispersed polymeric hydrophilic nanoparticles**

Verfahren zur Herstellung von hochmonodispersierten Polymeren hydrophiler Nanopartikel

Procédé pour la préparation de nanoparticules hydrophiles polymères hautement monodispersés

(84) Designated Contracting States:
**BE CH DE FR GB IT LI NL**

(43) Date of publication of application:
**11.11.1998   Bulletin 1998/46**

(73) Proprietors:
- **Maitra, Amarnath**
  **Delhi 110 007 (IN)**
- **Ghosh, P.K.**
  **Janakpuri,**
  **New Delhi 110 058 (IN)**
- **De K, Tapas**
  **Delhi 110 007 (IN)**
- **Sahoo, Sanjeeb Kumar**
  **Delhi 110 007 (IN)**

(72) Inventors:
- **Maitra, Amarnath**
  **Delhi 110 007 (IN)**
- **Ghosh, P.K.**
  **Janakpuri,**
  **New Delhi 110 058 (IN)**
- **De K, Tapas**
  **Delhi 110 007 (IN)**
- **Sahoo, Sanjeeb Kumar**
  **Delhi 110 007 (IN)**

(74) Representative: **O'Brien, Niall James et al**
**Urquhart-Dykes & Lord LLP**
**30 Welbeck Street**
**London W1G 8ER (GB)**

(56) References cited:
**FR-A- 2 208 716**

- **"Encyclopedia of polymer science and engineering Vol. 10 p.20,21" 1988, JOHN WILEY *a SONS , N.Y.**

## Description

This invention relates to a process for the preparation of highly monodispersed polymeric hydrophilic nanoparticles with or without target molecules encapsulated therein and having sizes of upto 100 nm and a high monodispersity.

PRIOR ART

Following an administration of a drug in a living system, the active substance is distributed throughout the body as a function of its physiochemical properties and molecular structure. The final amount of drug reaching its target site may only be a small fraction of the administered dose. Accumulation of drug at the non-targeted site may lead to adverse effect and undesirable side reactions. Therefore, targeting of drug to specific body sites is necessary.

One way of modifying the biodistribution of drugs in the body is to entrap them in ultrafine drug carriers. Among these carriers, liposomes, nanoparticles and pharmacosomes have been extensively studied. The use of liposomes as drug targeting agents is found to be limited due mainly to the problems of low entrapment efficiency, drug instability, rapid drug leakage and poor storage stability. With the aim of overcoming these problems, the production of polymeric nanoparticles has been investigated since the last two decades. Nanoparticles are defined as solid colloidal particles ranging in size from about 10nm to 1000nm.

A large number of studies have been reported on recent advances in drug targeting possibilities and sustained release action with nanoparticles encapsulating drugs. In vivo studies have also been reported with special attention to the reticuloendothelial system (RES). Some in vivo studies concerning nanoparticles administration by oral and ocular routes have also been reported in the literature with respect to the possible improvements of bioavailability. These polymeric nanoparticles should be non-antigenic, biocompatible and biodegradable.

The important characteristics of the particles used for targeting at specific body sites were found to be influenced mainly by two factors: (i) the size of the nanoparticles and (ii) the surface characteristics of the nanoparticles.

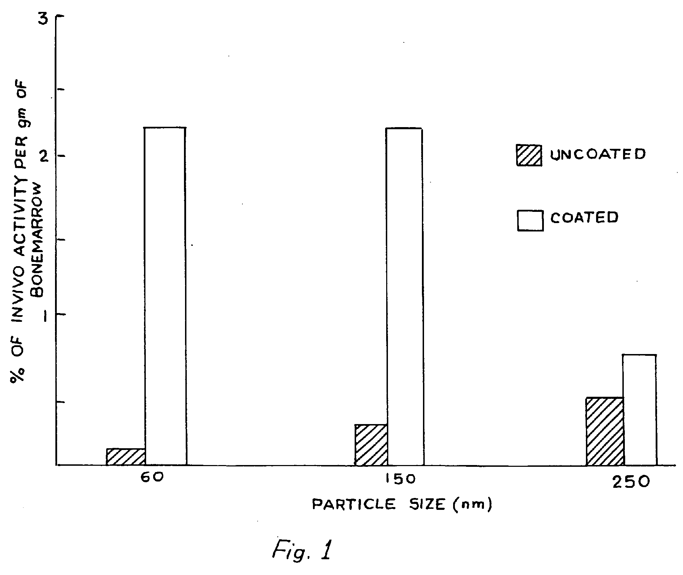

Particles smaller than 7 $\mu$m and specially nanoparticles are not filtered in the lung and their biodistribution is dependent on their interaction with reticuloendothelial system (RES). Biodegradable nanoparticles are mainly taken up by the Kupffer cells in the liver while small amount of these particles go to macrophages in spleen and bone marrow. Bone marrow uptake and targeting at other sites can be modified drastically by reducing the particle size. Nanoparticles of 200nm diameter and above have biodistribution dependent on their interaction with RES. The distribution, however, can be reversed if the particle size is made much smaller (ie. below 100nm) and particle surfaces are made hydrophilic. As an example, it has been found that if these particles are separated into three size ranges -60nm, 150nm and 250nm and their surfaces are rendered hydrophilic by adsorbing poloxamer type of surfactants, then the small sized particles with maximum surface hydrophilicity are mostly taken up by cells other than Kupffer cells and as shown in Fig.1 of the accompanying drawings. Specifically, Fig.1 shows size dependent uptake of nanoparticles, uncoated and coated with poloxamer surfactant by bone marrow. These small particles in the blood serum do not adsorb serum protein through opsonisation and as a result, their circulation time in blood is considerably increased. Hydrophobic particles are removed from the circulation very rapidly due to opsonisation. Nanometer sized particles with hydrophilic surface remain in blood for longer period of time so that targeting at specific sites may be facilitated.

At present, nanoparticles for drug encapsulation are prepared by methods involving either polymerisation of dispersed monomers or a dispersion of preformed polymers in emulsion in presence of desired drug. The methods known in the art for the preparation of nanoparticles are (i) dispersion polymerisation method, (ii) emulsion polymerisation method, (iii) dispersion of synthetic polymer nanospheres in emulsion, and (iv) interfacial polymerisation technique. In all these methods, emulsions of oil-in-water are used and the polymer is formed or dissolved in the oil phase.

As a result, the polymeric materials are always hydrophobic because they are to be soluble in oil and the particles formed are nanoparticles of larger size (ie. above 100nm) because the average size of the emulsion droplets is mostly 100nm and above diameter. Moreover, since the emulsion droplets are highly polydispersed, the nanoparticles formed have broad spectrum size range and these are also highly polydispersed. Thus, in such known processes (i) one cannot prepare nanoparticles of subcolloidal size and (ii) the emulsion medium demands that the polymeric materials should be hydrophobic

French Patent No 2 208 716 discloses a process for the production of particulate compositions using emulsion polymerisation. The particulate compositions may contain an active material such as a medicament, a catalyst or a dye.

OBJECTS OF THE INVENTION

An object of this invention is to propose a novel process for the preparation of highly monodispersed polymeric nanoparticles with or without targeted materials and having a size of upto 100nm with a high monodispersity.

A further object of this invention is to propose a process for the preparation of said polymeric nanoparticles capable of being modulated to required sizes.

Another object of this invention is to propose a process for the preparation of said highly monodispersed polymeric nanoparticles of subcolloidal size with or without targeted materials.

Still another object of this invention is to propose a process for the preparation of said hydrophilic polymeric nanoparticles.

A further object of this invention is to propose a process for the preparation of said highly monodispersed drug loaded polymeric nanoparticles dispersed in aqueous buffer and free of any toxic material.

A still further object of this invention is to propose a process for the preparation of highly monodispersed drug loaded polymeric nanoparticles of hydrophilic nature which obviates the disadvantages associated with those of the prior art.

Yet another object of this invention is to propose a process for the insertion and loading of target drug/target substance in nanoparticles to secure them from outer intervention in vivo or cell culture invitro till they are exposed at the target site within the cell.

## DESCRIPTION OF INVENTION

According to this invention, there is provided a process for the preparation of highly monodispersed polymeric hydrophilic nanoparticles with or without targeted materials having a size of upto 100nm with a high monodispersity comprising in the steps of :

        (i) dissolving a surfactant in oil to obtain reverse micelles;
        (ii) adding an aqueous solution of a monomer or preformed polymer to said reverse micelles and a cross-linking agent, initiator and drug or a target substance, if required;
        (iii) subjecting such a mixture to the step of polymerization;
        (iv) drying the polymerized reaction product for removal of solvent to obtain dry nanoparticles and surfactant;
        (v) dispersing the dry mass in aqueous buffer; and
        (vi) separating the surfactant and other toxic materials therefrom.

In accordance with this invention, the aqueous core of a reverse micellar droplet is used as a nanoreactor for the preparation of nanoparticles. The sizes of the aqueous core of such droplets are in the range of 1nm-10nm. The size of the particles which are formed primarily inside these droplets are larger than the size of the aqueous core of the droplets. Moreover, since the polymerisation takes place in an aqueous medium, polymers with surface hydrophilic properties are obtained by this invention. Therefore, using reverse micellar method of the present invention, it is possible to prepare very small size nanoparticles with hydrophilic surface so that their opsonisation as well as uptake by RES is substantially minimized. High monodispersity of the particles is possible because reverse micellar droplets in which the polymeric reactions are carried out are highly monodispersed.

The aqueous phase is regulated in such manner so as to keep the entire mixture in an optically transparent micro emulsion phase. The range of the aqueous phase cannot be defined as this would depend on factors such as the monomer, surfactant or polarity of oil, and the only factor is that the system is in an optically transparent micro emulsion phase.

In accordance with the present invention, the nanoparticles have a size range of upto 100nm, preferably a size of upto 10nm to 100nm.

In accordance with this invention, the aqueous core of a reverse micellar droplet is effectively used as nanoreactor to prepare ultrafine nanoparticles and to encapsulate the drugs (normally water soluble chemicals of maximum size upto that of 100-200 k Dalton protein. By the process of the present invention, extremely small particles of size of greater uniformity and down to about 10nm diameter has been achieved.

The surfactant, sodium bis ethylhexylsulphosuccinate, or Aerosol OT (ie. AOT) is dissolved in n-hexane to prepare reverse micelles. To the AOT solution in hexane (0.01M to 0.1M of AOT in hexane), aqueous solutions of monomer or preformed polymer, cross-linking agent, initiator and drug are added and the polymerisation is done in presence of nitrogen gas. Additional amount of water may be added in order to get nanoparticles of larger size. The maximum amount of drug that can be dissolved in reverse micelles varies from drug to drug and has to be determined by gradually increasing the amount of drug till the clear microemulsion is transformed into translucent solution. All the stock solutions are prepared in phosphate buffer and the contents swirled vigorously in order to ensure the transparency of the solution. The reaction mixture is purged with nitrogen gas. Polymerisation is done in nitrogen atmosphere. The solvent n-Hexane is then evaporated out at a temperature, for example, of 35°C using rotary evaporator under low pressure when transparent dry mass is obtained. The material is dispersed in water and to it CaC12 solution is added, drop by drop till all the calcium salt of diethylhexylsulphosuccinate (Ca(DEHSS)2 from AOT) is precipitated. The mixture is then subjected to centrifugation, for example, at 15,000 rpm for 10 mins.. The supernatant is decanted off which contains nanoparticles containing encapsulated drug. Some nanoparticles remain absorbed in the cake of the precipitate.

For complete recovery of the nanoparticles from the precipitated calcium (DEHSS)2, the latter is dissolved in n-hexane and the nanoparticles extracted with water. The aqueous dispersion is immediately dialysed through, for example, 12,000 cut off dialysis membrane for about one hour and the liquid ly-ophilised to dry powder and stored at low temperature till further use.

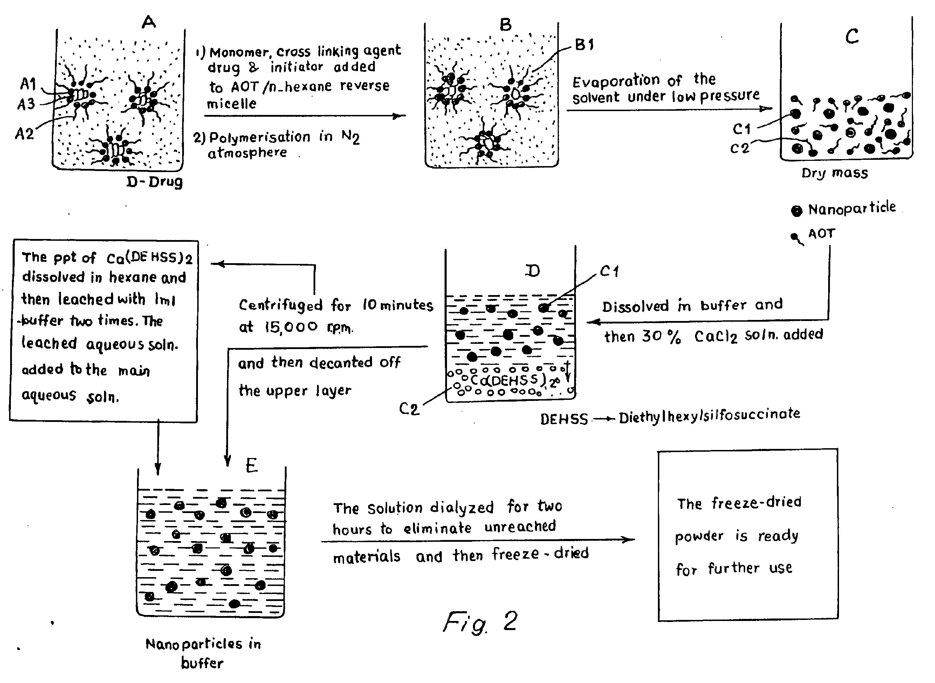

Reference is now made to Fig.2 of the accompanying drawings which illustrates the flow diagram for the preparation of nanoparticles using microemulsion. Step A shows a reverse micellar droplet A1, prepared by dissolving the surfactant in oil. Thus, when a surfactant is dissolved in oil, then the hydrophobic constituent tails A2 would remain in contact with oil and an inner core A3 would comprise of hydrophilic constituents. When water is added to a solution containing reverse micelle A1 of as the hydrophilic constituent is soluble in water, water is attracted to the hydrophilic domain or core A3. A monomer cross-linking agent, the required drug and initiator is added to the reverse micelle A1. As the aforesaid constituents are hydrophilic in nature, such constituents go to the core A3. Polymerization is carried out in nitrogen atmosphere to form a polymer B1 and encapsulated drug as shown in step B. In step B, an evaporation is effected under low pressure for removal of the solvent. Step C illustrates the dried mass to consist of nanoparticles CI, and surfactant C2. The dried mass is dissolved in phosphate buffer and then 30% CaCl2 added thereto drop by drop in step D to precipitate the surfactant as calcium diethylhexylsulfosuccinate (DEHSS). Step D illustrates the nanoparticles C1 and calcium DEHSS. The solution of step D is centrifuged at step E to obtain clear nanoparticles dispersed in buffer and the precipitate of Ca(DEHSS)2.

The cake of Ca(DEHSS)2 may contain some absorbed nanoparticles which can be recovered by dissolving the cake in hexane and leaching the nanoparticles by buffer 2 to 3 times. The leaching solutions are collected along with solution E. Such a buffer solution containing nanoparticles may still contain certain unreacted or toxic materials which are removed by dialyzing the solution for two hours and then freeze dried.

0.01 to 0.1M AOT in n-hexane is used. Vinylpyrrolidone (VP) or mixture of vinylpyrrolidone and polyethyleneglycolfumarate (PEGF) are used as monomers as they form water soluble hydrogels on polymerisation and are highly biocompatible. Another suitable but antigenic polymer which has been used is bovine serum albumin. Other suitable water soluble hydrogels and biocompatible materials can be used for nanoparticle formation. In case of hydrogels, the cross-linking is done with N,N methylene bis acrylamide (MBA) whereas albumin is crosslinked by glutaraldehyde. In case of polyvinylpyrrolidine (PVP) crosslinked with MBA, the amount of monomer used is, for example, about 50 w% of AOT, and the amount of cross-linking agent (MBA) used is 1.2% w/w of the polymer. Such a composition has maximum shelf life and retention of drug by nanoparticles of this composition is also maximum. Loading of drug should be between 1% to 10% w/w of the polymer according to the solubility of the drug in the micellar system but it can also be increased if the solubility of the drug in reverse micelles is high.

The following examples are given by way of illustration of the present invention and should not be construed to limit the scope of the present invention.

Example I : Preparation of an antigen loaded polyvinylpyrrolidone nanoparticles.

An antigen, from Aspergilus fumigatus, has been used as a drug for encapsulation. In a 40ml of 0.03M AOT solution in hexane, 140μl of freshly distilled pure vinylpyrrolidone 35μl of N,N methylene bis arylamide (0.49mg/ml), 20μl of 1% ferrous ammonium sulphate solution, 40μl of 11.2% aqueous solution of tetramethylethylenediamine (TMED), 10μl of 5% potassium persulphate as initiator and 180μl of antigen (antigen =16mg/ml) were added. The amount of excess buffer to be added in reverse micelles was governed by the desired size of the nanoparticles to be prepared. The volume of the excess buffer can be carried from zero to maximum amount upto which microemulsion formation is possible and no phase separation takes place. The solution was homogeneous and optically transparent. Polymerisation was done in presence of N2 gas at 30°C for 8 hours in a thermostatic bath with continuous stirring. The nanoparticles of polyvinylpyrrolidone containing encapsulated drug would be formed. The solvent was evaporated off in a rotary vacuum evaporator and the dry mass was resuspended in 5ml of water. Calculated amount of 30% CaCl2 solution was added drop by drop to precipitate AOT as calcium salt bisethylhexysulphosuccinate. The centrifuged aqueous solution contains nanoparticles which was homogeneous and almost transparent. The cake of calcium DEHSS after centrifugation contains some amount of nanoparticles absorbed in it. It was dissolved in 10ml of n-hexane and the hexane solution was washed 2-3 times each time with 1ml water. The phase separated clear aqueous layer was drained out and was collected with the original filtrate. The total aqueous dispersion of nanoparticles was then dialysed (12,000 cut off membrane) for about 2 hours against water and the dialysed solution was lyophilised immediately to dry powder for subsequent use. The sample should be free from AOT, monomer, cross-linking agent and perdisulphate. Any trace amount of unreacted materials and surfactant could be detected through HPLC. Perdisulphate was detected chemically using starch iodide solution and the presence of AOT was tested as follows :

To a 1mg/ml solution of dry powder, a drop of methylene blue dye was added. The solution was then mixed with 1ml of n-hexane thoroughly and was kept for phase separation. The hexane layer was then tested spectrophotometrically at 580nm for the presence of the dye.

Example II : The nanoparticles from polyethyleneglycolfumarate were prepared as follows :

5g of polyethylene glycol 600, 0.9g of fumaric acid and 1.22 mg of hydroquinone were mixed together and heated at 190°C for 7-8 hours in a 100ml 3-necked flask equipped with a thermometer, refluxing condenser and a nitrogen inlet. The product was of greenish yellow viscous liquid at room temperature.

In a 40ml of 0.06m AOT n-hexane the following components were added. 100$\mu$l of polyethyleneglycol fumarate (0.186g/ml), 10$\mu$l of freshly distilled vinylpyrrolidone, 10$\mu$l of N,N methylene bis acrylamide (0.049g/ml), 10$\mu$l of 0.5% ferrous ammonium sulphate, 20$\mu$l of 11.2% TMED and 10$\mu$l or 20$\mu$l, as the case may be of fluorescence isothiocyanate-dextran (FITC-dextran) of mol. wt. 16KD (160mg/ml). 0-200 $\mu$l of buffer depending on the size of the droplet were added.

In the above solution, N2 gas was passed for 30 mins. and then 10$\mu$l of 5% potassium perdisulphate was added as initiator with vigorous stirring. Thereafter, the nitrogen gas was passed through the solution for another six hours at 30°C.

The nanoparticles were recovered from the aqueous solution following the same method as described earlier in the case of polyvinylpyrrolidone particles.

Example III : Preparation of Bovine Serum Albumin-glytaraldehyde nanoparticles.

In a 40 ml of 0.06m AOT in n-hexane 200$\mu$l bovine serum albumin (100mg/ml) and 0-600$\mu$l water depending on the desired size of the micellar droplets were added. The mixture was thoroughly stirred at room temperature till a transparent microemulsion was formed. To the well stirred solution, 20$\mu$l 5% glutaraldehyde was added and the stirring was continued for another half an hour when the nanoparticles were formed. The aqueous solution of the nanoparticles were prepared from the AOT solution following the method as described in the case of polyvinylpyrrolidone above.

The nanoparticles were characterised as follows :

The entrapment efficiency of the FITC-dextran dye in polyvinylpyrrolidone nanoparticles was determined as follows : The aqueous extract including the repeated washings were collected and was made up to the volume of 10ml. 500 $\mu$l of the solution was filtered through 100KD membrane filter and 2 $\mu$d phosphate buffer was added. The absorbance of the solution was measured at 493nm. The absorbance of the same concentration of free FITC in phosphate buffer was measured. From the difference in absorbances, the entrapment efficiency was calculated and the values as shown in the table was found to be in the range of 39-44% irrespective of the size of the nanoparticles.

| Size of the PVP particles (nm) | Entrapment Efficiency (%) |
| --- | --- |
| 21 | 40 |
| 26 | 44 |
| 31 | 42 |
| 34 | 40 |
| 52 | 39 |
| 96 | 40 |

The size of the nanoparticles was determined by laser light scattering measurements.

Dynamic laser light scattering measurements for determining the size of the nanoparticles were performed using Brookhaven 9000 instrument with BI200SM goniometer. Argon ion air cooled laser was operated at 488nm as a light source. The time dependence of the intensity autocorrelation function of the scattered intensity was derived by using 128 channel digital correlator. Intensity correlation data was processed by using the method of cumulants. The translational diffusion coefficient ($\sigma$T) of the particles dispersed in aqueous buffer was obtained from a non-linear least square fit of the correlation curve using the decay equation. From the value of the translational diffusion coefficient, the average of hydrodynamic diameter Dh of the scattering particles was calculated by Stokes-Einstein relationship

$$D_h = kT/3\tilde{\Delta}\eta\sigma\ T$$

where k is Boltzmen constant, $\eta$ is the viscosity of the solvent at an absolute temperature T.

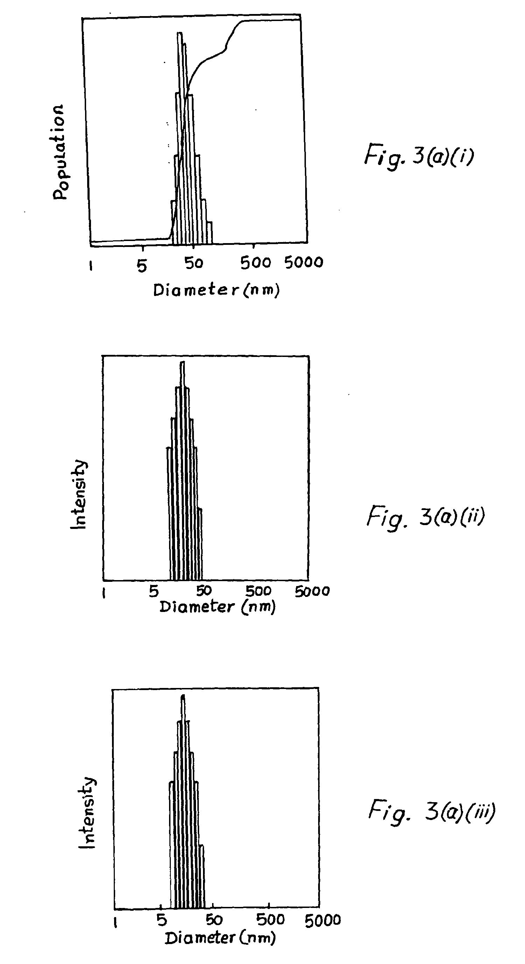

The size of the drug loaded nanoparticles of polyvinylpyrrolidone, polyethyleneglycolfumarate and bovine serum albumin were determined and representative spectra for each type are shown in the Figure 3(a). Fig.3(a) (i to iii) show

(i) nanoparticles made of polyethylene glycol fumarate containing FITC-Dextran

(ii) nanoparticles made of polyvinylpyrrolidone containing FITC-Dextran,

(iii) nanoparticles made of bovine serum albumin crosslinked with glutaraldehyde.

Fig.3(b) shows the variation of particle size with the change of size of the microemulsion droplets. Interestingly, the size of the polyvinylpyrrolidone nanoparticles increases exponentially with the increase of droplet size whereas the same remain more or less constant in case of bovine serum albumin-glutaraldehyde particles.

In vitro release kinetic studies :

A known amount of lyophilised nanoparticles encapsulating FITC-dextran was suspeded in 10ml of phosphate buffer saline in 50ml polypropylene tubes. The tubes were placed in water bath maintained at 37°C. At predetermined intervals, a volume of 500µl taken from each tube was passed through a 100KD filter (Millipore UFP2THK24) which retained the nanoparticles and the free dye came out in the filtrate. The dye concentration in the filtrate was determined spectrophotometrically.

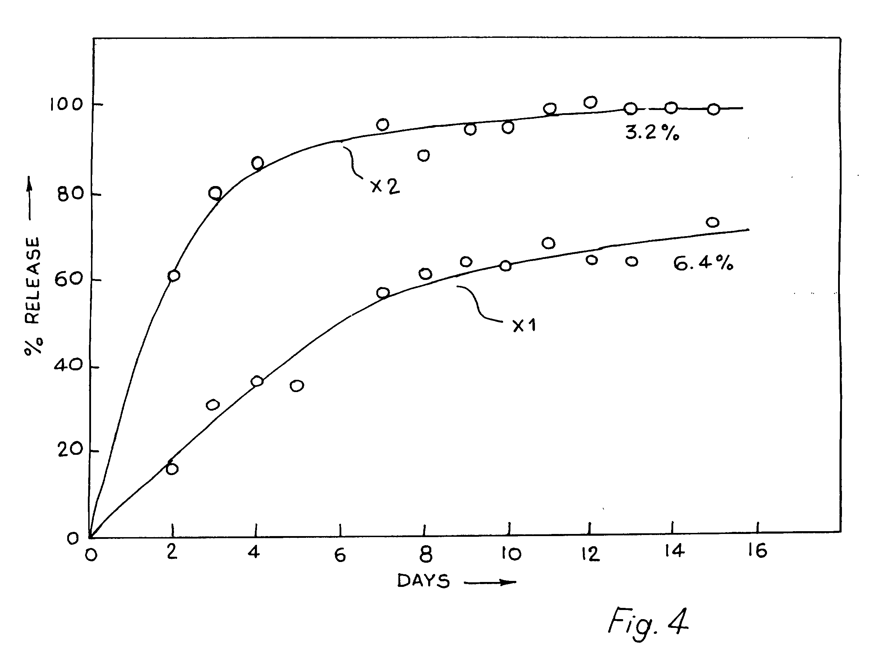

The results are shown in Fig. 4, which illustrates the release of FITC-dextran dye from polyethylene glycol fumarate particles of different loading, and where curve X1 shows a 6.4% loading and curve X2 is 3.2% loading of dye.

In vivo antibody response in mice serum by injecting antigen encapsulated nanoparticles.

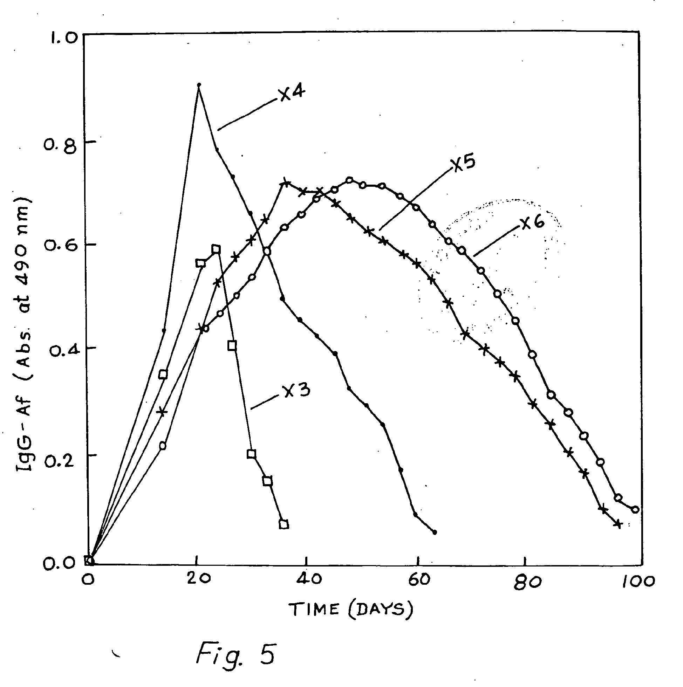

Mice were injected subcutaneously three times at an interval of 7 days with PVP nanoparticles containing 300µg of Aspergillius fumigatus antigen entrapped in the PVP nanoparticles and suspended in 100µl of normal saline. Each group contains five animals, three of which received the antigen entrapped in nanoparticles, one received free antigen (300µg) and the control received empty nanoparticles suspended in normal saline. Mice were bled at predetermined intervals and the amount of Aspergilus fumigatus specific antibody in the mice serum was assayed using indirect ELISA assay. The results are shown in Fig.5, which illustrates specific antibody response.

Specific antibody response of antigen of Apergilus fumigatus entrapped in polyvinylpyrrolidone nanoparticles at different amount of cross-lining agent with curves X3,X4,X5 and X6 having 0%, 0.3%, 0.6% and 1.2% of cross-linking agent respectively.

## Claims

1. A process for the preparation of nearly monodispersed polymeric hydrophilic nanoparticles with or without targeted materials having an average size of up to 100 nm comprising the steps of:

(i) dissolving a surfactant in oil to obtain reverse micelles;
(ii) adding an aqueous solution of a monomer or preformed polymer to said reverse micelles, a cross-linking agent, an initiator, and if required a drug or a target substance, wherein the aqueous phase is regulated such that the mixture is in an optically transparent micro emulsion phase;
(iii) subjecting such a mixture to the step of polymerization in the instance of said monomer solution and to a cross-linking reaction in the instance of said preformed polymer;
(iv) drying the polymerized reaction product for removal of solvent to obtain dry nanoparticles and surfactant;
(v) dispersing the dry mass in aqueous buffer;
(vi) treating the resulting mixture with calcium chloride to remove the surfactant; and
(vii) separating the other toxic materials therefrom.

2. A process as claimed in Claim 1 wherein said nanoparticles have an average size of 10nm to 100nm.

3. A process as claimed in Claim 1 wherein said monomers and/or preformed polymers are biocompatible and non-antigenic materials such as vinylpyrrolidone or a mixture of vinylpyrrolidone and polyethyleneglycolfumarate, or their polymers such as polyvinylpyrrolidone or co-polmyer of polyvinylpyrrolidone and polyethyleneglycolfumarate.

4. A process as claimed in Claim 1 wherein said polymers are biocompatible but antigenic such as bovine serum albumin.

5. A process as claimed in Claim 1 wherein said crosslinking agent is N,N methylene-bis acrylamide (MBA) or glutaraldehyde.

6. A process as claimed in Claim 1 wherein the said initiators are water soluble perdisulphate salts like ammonium perdisulphate and the activator is tetramethyl ethylene diamine (TMED).

**7.** A process as claimed in Claim 1 wherein 1% to 10% target substance by weight of the polymeric substance is encapsulated into said nanoparticles.

**8.** A process as claimed in Claim 1 wherein 0.01M to 0.1M of surfactants such ass sodium bis etyhyl hexyl sulphosuccinate are used for reverse micelles preparation.

**9.** A process as claimed in claim 1 wherein said hydrocarbons are alkanes such as n-hexane.

**10.** A process as claimed in claim 1 wherein the nanoparticles dispersed in aqueous buffer is dialysed to remove the unreacted materials from the buffer.

**11.** A process as claimed in claim 1 wherein the dispersed nanoparticles after dialysis is lyophilised and preserved.

**Patentansprüche**

**1.** Verfahren zur Herstellung von nahezu monodispersen polymeren hydrophilen Nanopartikeln mit oder ohne Targetmaterialien mit einer durchschnittlichen Größe von bis zu 100 nm, umfassend die Schritte von:

(i) Auflösen eines Tensids in Öl zum Erhalt reverser Micellen;
(ii) Zufügen einer wässrigen Lösung von einem Monomer oder vorgebildeten Polymer zu den reversen Micellen, eines Vernetzungsmittels, eines Initiators und gegebenenfalls eines Arzneimittels oder einer Targetsubstanz, worin die wässrige Phase dergestalt reguliert ist, dass sich das Gemisch in einer optisch transparenten Mikroemulsionsphase befindet;
(iii) Aussetzen eines solchen Gemischs dem Polymerisationsschritt im Fall der Monomerlösung und einer Vernetzungsreaktion im Fall des vorgebildeten Polymers;
(iv) Trocknen des polymerisierten Reaktionsprodukts zur Entfernung des Lösungsmittels zum Erhalt trockener Nanopartikel und von Tensid;
(v) Dispergieren der Trockenmasse in wässrigem Puffer;
(vi) Behandeln der sich ergebenden Mischung mit Calciumchlorid zur Entfernung des Tensids; und
(vii) Trennen der anderen toxischen Materialien davon.

**2.** Verfahren nach Anspruch 1, worin die Nanopartikel eine durchschnittliche Größe von 10 nm bis 100 nm aufweisen.

**3.** Verfahren nach Anspruch 1, worin die Monomere und/oder vorgebildeten Polymere biokompatible und nicht antigene Materialien, wie zum Beispiel Vinylpyrrolidon oder ein Gemisch aus Vinylpyrrolidon und Polyethylenglykolfumarat, oder ihre Polymere, wie zum. Beispiel Polyvinylpyrrolidon, oder das Copolymer von Polyvinylpyrrolidon und Polyethylenglykolfumarat, darstellen.

**4.** Verfahren nach Anspruch 1, worin die Polymere biokompatibel, aber antigen sind, wie zum Beispiel Rinderserumalbumin.

**5.** Verfahren nach Anspruch 1, worin das Vernetzungsmittel N,N-Methylen-bis-acrylamid (MBA) oder Glutaraldehyd darstellt.

**6.** Verfahren nach Anspruch 1, worin die Initiatoren wasserlösliche Peroxodisulfatsalze, wie zum Beispiel Ammoniumperoxodisulfat darstellen und der Aktivator Tetramethylethylendiamin (TMED) darstellt.

**7.** Verfahren nach Anspruch 1, worin 1 bis 10 Gew.-% Targetsubstanz der polymeren Substanz in den Nanopartikeln verkapselt ist.

**8.** Verfahren nach Anspruch 1, worin 0,01 M bis 0,1 M Tenside, wie zum Beispiel Natrium-bis-ethyl-hexylsulfosuccinat, zur Herstellung reverser Micellen verwendet werden.

**9.** Verfahren nach Anspruch 1, worin die Kohlenwasserstoffe Alkane, wie zum Beispiel n-Hexan, darstellen.

**10.** Verfahren nach Anspruch 1, worin die Nanopartikel, die im wässrigen Puffer dispergiert sind, zur Entfernung der nicht zur Reaktion gebrachten Materialien aus dem Puffer dialysiert werden.

**11.** Verfahren nach Anspruch 1, worin die dispergierten Nanopartikel nach der Dialyse lyophilisiert und konserviert werden.


**Revendications**

**1.** Procédé de préparation de nanoparticules hydrophiles polymères presque monodispersées, avec ou sans matériaux ciblés, possédant une granulométrie moyenne allant jusqu'à 100 nm, comprenant les étapes :

(i) de dissolution d'un agent tensio-actif dans une huile pour obtenir des micelles inverses ;

(ii) d'addition d'une solution aqueuse d'un monomère ou d'un polymère préformé auxdites micelles inverses, d'un agent de réticulation, d'un initiateur, et si nécessaire d'une substance médicamenteuse ou cible, dans laquelle la phase aqueuse est contrôlée de sorte que le mélange soit sous forme d'une phase de type micro-émulsion optiquement transparente ;

(iii) de soumission de ce mélange à une étape de polymérisation dans le cas de ladite solution de monomère et à une réaction de réticulation dans le cas dudit polymère préformé ;

(iv) de séchage du produit de réaction polymérisé pour éliminer le solvant afin d'obtenir les nanoparticules et l'agent tensio-actif secs ;

(v) de dispersion de la masse sèche dans un tampon aqueux;

(vi) de traitement du mélange résultant au chlorure de calcium pour éliminer l'agent tensio-actif ; et

(vii) de séparation des autres matériaux toxiques de celui-ci.

**2.** Procédé selon la revendication 1, dans lequel lesdites nanoparticules possèdent une granulométrie moyenne de 10 nm à 100 nm.

**3.** Procédé selon la revendication 1, dans lequel lesdits monomères et/ou polymères préformés sont des matériaux biocompatibles et non antigènes, tels que la vinylpyrrolidone ou un mélange de vinylpyrrolidone et de poly(fumarate d'éthylèneglycol), ou leurs polymères tels que le polyvinylpyrrolidone ou un copolymère de polyvinylpyrrolidone et de poly(fumarate d'éthylèneglycol).

**4.** Procédé selon la revendication 1, dans lequel lesdits polymères sont biocompatibles mais antigènes, tels que l'albumine de sérum bovin.

**5.** Procédé selon la revendication 1, dans lequel ledit agent de réticulation est le N,N-méthylène-bis acrylamide (MBA) ou le glutaraldéhyde.

**6.** Procédé selon la revendication 1, dans lequel lesdits initiateurs sont des sels de type perdisulfate solubles dans l'eau, tels que le perdisulfate d'ammonium, et l'activateur est la tétraméthyléthylènediamine (TMED).

**7.** Procédé selon la revendication 1, dans lequel la substance cible est encapsulée dans lesdites nanoparticules à hauteur de 1 % à 10% en poids de la substance polymère.

**8.** Procédé selon la revendication 1, dans lequel des agents tensioactifs, tels que le bis-étyhylhexyl sulfosuccinate de sodium 0,01 M à 0,1 M, sont utilisés pour la préparation des micelles inverses.

**9.** Procédé selon la revendication 1, dans lequel lesdits hydrocarbures sont des alcanes tels que le n-hexane.

**10.** Procédé selon la revendication 1, dans lequel les nanoparticules dispersées dans le tampon aqueux sont dialysées pour éliminer les matériaux du tampon n'ayant pas réagi.

**11.** Procédé selon la revendication 1, dans lequel les nanoparticules dispersées après la dialyse sont lyophilisées et conservées.

Fig. 1

**A**

A1
A3
A2

D-Drug

1) Monomer, cross linking agent
drug & initiator added
to AOT/n-hexane reverse
micelle

2) Polymerisation in N₂
atmosphere

**B**

B1

Evaporation of the
solvent under low pressure

**C**

C1
C2

Dry mass

● Nanoparticle

ᕋ AOT

The ppt of Ca(DEHSS)₂
dissolved in hexane and
then leached with 1ml
-buffer two times. The
leached aqueous soln.
added to the main
aqueous soln.

Centrifuged for 10 minutes
at 15,000 r.p.m.

and then decanted off
the upper layer

**D**

C1

Dissolved in buffer and
then 30% CaCl₂ soln. added

Ca(DEHSS)₂

C2

DEHSS ⟶ Diethylhexylsilfosuccinate

**E**

Nanoparticles in
buffer

The solution dialyzed for two
hours to eliminate unreached
materials and then freeze-dried

The freeze-dried
powder is ready
for further use

*Fig. 2*

EP 0 877 033 B1

Fig. 3(a)(i)

Fig. 3(a)(ii)

Fig. 3(a)(iii)

Fig. 4

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2208716